Europäisches Patentamt

European Patent Office

Office européen des brevets

(11) Publication number : **0 505 181 A1**

# EUROPEAN PATENT APPLICATION

(21) Application number : **92302373.3**

(22) Date of filing : **19.03.92**

(51) Int. Cl.⁵ : **A61K 31/70**

(30) Priority : **20.03.91 GB 9105899**

(43) Date of publication of application :
**23.09.92 Bulletin 92/39**

(84) Designated Contracting States :
**AT BE CH DE DK ES FR GB GR IT LI LU MC NL PT SE**

(71) Applicant : **THE WELLCOME FOUNDATION LIMITED**
**Unicorn House 160 Euston Road**
**London NW1 2BP (GB)**

(72) Inventor : **Rideout, Janet Litster**
**3101 Morningside Drive**
**Raleigh, North Carolina 27607 (US)**
Inventor : **Averett, Devron Randolph**
**2608 Lochmore Drive**
**Raleigh, North Carolina 27608 (US)**
Inventor : **Freeman, George Andrew**
**3900 Inland Court**
**Raleigh, North Carolina 27606 (US)**

(74) Representative : **Garrett, Michael et al**
**The Wellcome Research Laboratories Group**
**Patents and Agreements Langley Court**
**Beckenham Kent BR3 3BS (GB)**

(54) **Use of 3'-azido-2',3'-dideoxyguanosine for the treatment of hepatitis B.**

(57) The present invention relates to the use of 9-(3-azido-2,3-dideoxy-β-D-erythro-pentofuranosyl) guanine or a salt, ester or other physiologically functional derivative thereof, in the manufacture of a medicament for the treatment or prophylaxis of hepatitis B viralinfections.

EP 0 505 181 A1

The present invention relates to the use of 9-(3-azido-2,3-dideoxy-β-D-erythro-pentofuranosyl)guanine, or a salt, ester or other physiologically functional derivative thereof, in the manufacture of a medicament for the treatment or prophylaxis of hepatitis B viral infections.

Hepatitis B virus (HBV) is a viral pathogen of major consequence worldwide. HBV is most common in Asian Countries, and prevalent in sub-Saharan Africa. The virus is etiologically associated with primary hepatocellular carcinoma and is thought to cause 80% of the world's liver cancer. In the United States more than ten thousand people are hospitalized for HBV illness each year, an average of 250 die with fulminant disease. The United States currently contains an estimated pool of 500,000 - 1 million infectious carriers. Chronic active hepatitis will develop in over 25% of carriers and often progresses to cirrhosis. It is estimated that 5000 people die from HBV related cirrhosis each year in the U.S.A. and that perhaps 1000 die from HBV-related liver cancer. Even when a universal HBV vaccine is in place, the need for effective anti-HBV compounds will continue. The large reservoir of persistently infected carriers, estimated at 220 million worldwide, will receive no benefit from vaccination and will continue at high risk for HBV induced liver disease. This carrier population serves as the source of infection of susceptible individuals perpetuating the instance of disease particularly in endemic areas or high risk groups such as IV drug abusers and homosexuals. Thus, there is a great need for effective antiviral agents, both to control the chronic infection and reduce progression to hepatocellular carcinoma.

Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease as outlined above . In "Viral Infections of Humans" (second edition, Ed., Evans, A.S. (1982) Plenum Publishing Corporation, New York), Chapter 12 describes in detail, the etiology of viral hepatitis infections.

In U.S. Patent 4,724,232 there is described 3'-azido-3'-deoxythymidine (which has the approved name zidovudine), its pharmaceutically acceptable derivatives and their use in the treatment of human retrovirus infections including AIDS and associated clinical conditions. Further 3'-azido nucleoside analogues are described in European Patent Specification 0 217 580.

European Patent Application 0 286 825 (US 4,880,782) discloses 9-(3-azido-2,3-dideoxy-β-D-erythro-pentofuranosyl)guanine and other 3'-azido nucleoside analogues for treating a human infected with a virus characterised by RNA dependent polymerase activity, in particular a human retroviral infection and especially a human immunodeficiency virus (HIV) infection.

We have now surprisingly found that 9-(3-azido-2,3-dideoxy-β-D-erythro-pentofuranosyl)guanine has potent activity against HBV.

The present invention therefore lies in the use of 9-(3-azido-2,3-dideoxy-β-D-erythro-pentofuranosyl)guanosine or a salt, ester or other physiologically functional derivatives thereof, in the manufacture of a medicament for the treatment or prophylaxis of hepatitis B viral infections.

Further aspects of the present invention include:-

(a) A method for the treatment, prophylaxis or prevention of the symptoms or effects of hepatitis B viral infections, in an infected subject (host), for example, a mammal including humans which comprises administering to said subject (host) a therapeutically effective non toxic amount of 9-(3-azido-2,3-dideoxy-β-D-erythro-pentofuranosyl)guanine or a salt, ester or other physiologically functional derivative thereof.

(b) Pharmaceutical formulations for use in the treatment or prophylaxis of hepatitis B viral infections containing 9-(3-azido-2,3-dideoxy-β-D-erythro-pentofuranosyl)guanine or a salt, ester or other physiologically functional derivative thereof.

As used herein the term "physiologically functional derivative" means any physiologically acceptable salt, ester or salt of such ester, of 9-(3-azido-2,3-dideoxy-β-D-erythro-pentofuranosyl)guanine or any other compound which upon administration to the recipient, is capable of providing (directly or indirectly) the parent compound or an active metabolite or residue thereof.

Preferred esters in accordance with the invention include carboxylic acid esters in which the non-carbonyl moiety of the carboxylic acid portion of the ester grouping is selected from straight or branched chain alkyl (e.g. methyl, n-propyl, t-butyl, n-butyl), cycloalkyl, alkoxyalkyl (e.g. methoxymethyl), arylalkyl (e.g. benzyl), aryloxyalkyl (e.g. phenoxymethyl), aryl (e.g. phenyl optionally substituted by halgen, $C_{1-4}$ alkyl or $C_{1-4}$ alkoxy); sulfonate esters such as alkyl- or alkylarylsulfonyl (e.g. methanesulfonyl); amino acid esters (e.g. L-valyl or L-isoleucyl); and mono- di- or tri-phosphate esters. The phosphate esters may be further esterified by, for example, a $C_{1-20}$ alcohol or reactive derivative thereof, or by a 2,3-di($C_{6-24}$)acyl glycerol.

With regard to the above-described esters unless otherwise specified, any alkyl moiety present advantageously contains 1 to 18 carbon atoms, particularly 1 to 4 carbon atoms. Any cycloalkyl moiety present in such esters advantageously contains from 3 to 6 carbon atoms. Any aryl moiety present in such esters advantageously comprises a phenyl group.

Any reference to any of the above compounds also includes a reference to a physiologically acceptable salt thereof.

Examples of physiologically acceptable salts according to the invention and physiologically acceptable derivatives thereof include base salts, e.g. derived form an appropriate base, such as alkali metal (e.g. sodium), alkaline earth metal (e.g. magnesium) salts, ammonium and $NX_4^+$ (wherein X is $C_{1-4}$ alkyl). Pharmaceutically acceptable acid addition salts include salts of organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids.

For therapeutic use, salts of 9-(3-azido-2,3-dideoxy-β-D-erythro-pentofuranosyl)guanine will be physiologically acceptable, i.e. they will be salts derived from a physiologically acceptable acid or base. However, salts of acids or bases which are not physiologically acceptable may also find use, for example, in the preparation or purification of a physiologically acceptable compound. All salts, whether or not derived from a physiologically acceptable acid or base, are within the scope of the present invention.

9-(3-azido-2,3-dideoxy-β-D-erythro-pentofuranosyl)guanine or a salt, ester or other physiologically functional derivative thereof may be employed alone or in combination with other therapeutic agents. Examples of such further therapeutic agents which are effective for the treatment of HBV infections include carbovir, oxathiolan nucleoside analogues such as cis-1-(2-(hydroxymethyl)-1,3-oxathiolan-5-yl)-cytosine (BCH-189) and interferons, for example, α interferon. The component compounds of such combination therapy may be administered simultaneously, in either separate or combined formulations, or at different times, for example, sequentially, such that a combined effect is achieved.

The amount of 9-(3-azido-2,3-dideoxy-β-D-erythro-pentofuranosyl)-guanine or salt, ester or other physiologically functional derivative thereof which is required in a medication to achieve the desired effect will depend on a number of factors, in particular the specific application, the nature of the particular compound used, the mode of administration and the condition, weight, age and sex of the patient and will ultimately be at the discretion of the attendant physician.

In general, a suitable dose for the treatment of hepatitis B viral infections is in the range of 0.5 to 120 mg per kilogram body weight of the recipient per day, preferably in the range of 1 to 90 mg per kilogram body weight per day and most preferably in the range 2 to 60 mg per kilogram body weight per day. An optimum dose is about 10mg per kilogram bodyweight per day (unless otherwise indicated all weights of active ingredients are calculated as the parent compound of formula (I), for salts and esters thereof the figures would be increased proportionately). The desired dose is preferably presented as two, three, four, five, six or more sub-doses administered at appropriate intervals throughout the day. These sub-doses may be administered in unit dosage forms, for example, containing 10 to 1500 mg, preferably 5 to 1000 mg, and most preferably 10 to 700 mg of active ingredient per unit dosage form. Alternatively, if the condition of the patient so requires, the dose may be administered as a continuous flow.

Ideally, the active ingredient should be administered to achieve peak plasma concentrations of the active compound of from about 0.25 to about 100 μM, preferably about 0.5 to 70 μM, most preferably about 1 to about 50 μM. This may be achieved, for example, by the intravenous injection of a 0.1 to 5% solution of the active ingredient, optionally in saline, or orally administered, for example, as a tablet, capsule, or syrup containing about 0.5 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

In the manufacture of a medicament according to the invention, hereinafter referred to as a "formulation", 9-(3-azido-2,3-dideoxy-β-D-erythro-pentofuranosyl)guanine or a salt, ester or other physiologically functional derivative thereof, also referred to herein as "active ingredient", is typically admixed with, inter alia, one or more acceptable carriers thereof and optionally other therapeutic agents. Each carrier must be "acceptable" in the sense of being compatible with the other ingredients of the formulation and not injurious to the patient. It is also possible for the active ingredient to be administered alone.

Formulations of the invention include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or suspension in an aqueous or non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose) surface-active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein using, for example hydroxypropylmethyl cellulose in varying proportions to provide the desired release profile. Tablets may optionally be provided with an enteric coating, to provide release in parts of the gut other than the stomach. This is particularly advantageous for purine nucleoside derivatives as such compounds are susceptible to acid hydrolysis.

Formulations suitable for oral use as described above may also include buffering agents designed to neutralize stomach acidity. Such buffers may be chosen from a variety of organic or inorganic agents such as weak acids or bases admixed with their conjugated salts.

A capsule may be made by filling a loose or compressed powder on an appropriate filling machine, optionally with one or more additives. Examples of suitable additives include binders such as povidone; gelatin, lubricants, inert diluents and disintegrants as for tablets. Capsules may also be formulated to contain pellets or discrete sub-units to provide slow or controlled release of the active ingredient. This can be achieved by extruding and spheronising a wet mixture of the drug plus an extrusion aid (for example microcrystalline cellulose) plus a diluent such as lactose. The spheroids thus produced can be coated with a semi-permeable membrane (for example ethyl cellulose, Eudragit WE30D) to produce sustained release properties.

An edible foam or whip formulation ideally comprises; 50-70% of an edible oil, particularly a vegetable oil, including corn oil, peanut oil, sunflower oil, olive oil and soybean oil; 2-10% of one or more surfactants particularly lecithin, polyols, polyol polymer esters including glyceryl fatty acid esters, polyglyceryl fatty acid esters (e.g. decaglycerol tetraoleate), or sorbitan fatty acid esters (e.g. sorbitan monostearate); 1-4% of a propellant which is suitable for ingestion, notably a compressed gas propellant especially nitrogen, nitrous oxide or carbon dioxide, or a gaseous hydrocarbon especially propane, butane or isobutane; 0.5-30% of one or more viscosity modifiers of particle size in the range 10-50 microns in diameter, particularly powdered sugars or colloidal silicon dioxide; and optionally 0.5-1% of one or more suitable, non-toxic colourings, flavourings or sweetners. The active ingredient is preferably present in such formulations in a concentration of 10-46%, advantageously 30%. An edible foam or whip formulation as described above may be prepared in a conventional manner, for example by mixing the edible oil, surfactant(s) and any other soluble ingredients, adding the viscosity modifier(s) and milling the mixture to form a uniform dispersion and suspension. The active ingredient is blended into the milled mixture until evenly dispersed. Finally, a metered quantity of propellant is incorporated to the mixture after said mixture has been measured into a suitable dispensing container.

Pharmaceutical formulations for topical administration according to the present invention may be formulated as an ointment, cream, suspension, lotion, powder, solution, paste, gel, spray, aerosol or oil. Alternatively, a formulation may comprise a dressing such as a bandage or adhesive plaster impregnated with active ingredients and optionally one or more excipients or diluents.

Compositions suitable for transdermal administration may be presented as discrete patches adapted to remain in intimate contact with the epidermis of the recipient for a prolonged period of time. Such patches suitably contain the active compound 1) in an optionally buffered, aqueous solution of 2) dissolved in an adhesive or 3) dispersed in a polymer. A suitable concentration of the active compound is about 1% to 35%, preferably about 3% to 15%. As one particular possibility, the active compound may be delivered from the patch by iontophoresis as generally described in Pharmaceutical Research, 3(6), 318 (1986).

For infections of the eye or other external tissues, for example mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base or as a water-in-oil base.

If desired, the aqueous phase of the cream base may include, for example, at least 40-45% w/w of a polyhydric alcohol, i.e. an alcohol having two or more hydroxyl groups such as propylene glycol, butane-1,3-diol, mannitol, sorbitol, glycerol and polyethylene glycol and mixtures thereof. The topical formulations may include a compound which enhances absorption or penetration of the active ingredient through the skin or other affected areas. Examples of such dermal penetration enhancers include dimethylsulphoxide and related analogues.

The oily phase of an emulsion formulation according to the invention may comprise merely an emulsifier (otherwise known as an emulgent), but desirably comprises a mixture of at least one emulsifier with a fat or an oil or with both a fat and an oil. Preferably, a hydrophilic emulsifier is included together with a lipophilic emulsifier which acts as a stablilizer. It is also preferred to include both an oil and a fat. Together, the emulsifer(s) with or without stabilizer(s) make up the so-called emulsifying wax, and the wax together with the oil and/or fat make up the so-called emulsifying ointment base which forms the oily phase of the cream formulations.

Emulgents and emulsion stabilizers suitable for use in the formulation of the present invention include Tween 60, Span 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. The cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent. The ingredient is preferably present in such formulations in a concentration of 0,5 to 20%, advantageously 0.5 to 10%, particularly about 1.5% w/w.

Formulations suitable for topical administration in the mouth include lozenges comprising the active ingredient in a flavoured material, usually sucrose and acacia or tragacanth; pastilles comprising the active ingredient in an inert material such as gelatin and glycerin, or sucrose and acacia; and mouth-washes comprising the active ingredient in a suitable liquid carrier.

Formulations for rectal administration may be presented as a suppository with a suitable base comprising for example cocoa butter or higher fatty alcohol (e.g. hard wax, European Pharmacopoeia) or triglycerides and saturated fatty acids (e.g. Witepsol).

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injections solutions which may contain anti-oxidants, buffers, bacteriostats and solutes which render the formulation isotonic with the blood of the intended recipient; and aqueous and non-aqueous sterile suspensions which may include suspending agents and thickening agents, as liposomes or other microparticulate systems which are designed to target the compounds to blood components or one or more organs. The formulations may be presented in unit-dose or multi-dose sealed containers, for example, ampules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose as herein above recited, or an appropriate fraction thereof, of an active ingredient.

It should be understood that in addition to the ingredients particularly mentioned above the formulations of this invention may include other agents conventional in the art having regard to the type of formulation in question, for example, those suitable for oral administration may include such further agents as sweeteners, thickeners and flavoring agents.

9-(3-azido-2,3-dideoxy-β-D-erythro-pentofuranosyl)guanine and salts, esters and other physiologically functional derivatives thereof, may be prepared by methods that are known in the art of nucleoside synthetic chemistry. Typically the methods are the same as or analogous to those described in US Patent 4,724,233.

9-(3-azido-2,3-dideoxy-β-D-erythro-pentofuranosyl)guanine may be prepared by the method of Imazawa and Eckstein (1978), J.Org.Chem., Vol 43, p3044-3048.

9-(3-azido-2,3-dideoxy-β-D-erythro-pentofuranosyl)guanine may be converted into a physiologically functional ester by reaction with an appropriate esterifying agent, for example, an acid halide or anhydride. 9-(3-azido-2,3-dideoxy-β-D-erythro-pentofuranosyl)guanine or esters thereof, may be converted into physiologically functional salts thereof in a conventional manner, for example, by treatment with an appropriate base. An ester or salt of 9-(3-azido-2,3-dideoxy-β-D-erythro-pentofuranosyl)guanine may be converted into the parent compound, for example, by hydrolysis.

For a better understanding of the invention, the following Examples are given by way of illustration.

## Example 1

### Preparation of 9-(3-azido-2.3-dideoxy-β-D-erythro-pentofuranosyl)-guanine

N-2-Palmitoylguanine (49.5g, 0.127 mol, prepared by the method of Kowollik et al., Tetrahedron Lett. 3863 (1969)) and 340 mL of 1,1,1,3,3,3,-hexamethyldisilazane and 10 mL of chlorotrimethylsilane were heated 5 h at reflux in a flame-dried 2L 3-necked round bottom flask equipped with reflux condenser, mechanical stirrer and nitrogen inlet. An additional 10 mL of chlorotrimethylsilane were added after the mixture was heated 90 minutes at reflux. The solution becomes homogeneous after 3.5 h at reflux. After standing overnight at room temperature most of the solvent was removed by distillation at 20 mmHg. Residual 1,1,1,3,3,3-hexamethyldisilazane was removed by evaporating the crude oil from 2 X 150 mL portions of toluene. The residue was cooled to room temperature, a solution of 5'-O-acetyl-3'-azido-3'-deoxythymidine (23.1g, 0.127 mol prepared by the method Kowollick et al . 1969, in 250 mL of acetonitrile (freshly distilled from $CaH_2$) was added followed immediately by the addition of 15.5 mL of trimethylsilyl trifluormethanesulfonate. The mixture was heated 3 h at reflux, cooled to room temperature and poured into a vigorously stirred mixture of 2 L of 10% $KHCO_3$ amd 2 L of ethyl acetate. The aqueous layer was extracted with an additional 3 X 750 mL portions of ethyl acetate and the combined organic extracts were concentrated by rotary evaporation to afford 16.2 grams of off-white solids. The solids were purified by flash chromatography starting with $CHCl_3$:EtOH (99:1) and slowly increasing the ethanol in the gradient by 1% every 2 liters. The appropriate fractions were concentrated by rotary evaporation to afford 2.5 g (8.2%) of 9-(3-azido,2,3-dideoxy-5-O-acetyl-β-D-erythro-pentofuranosyl)N-2-palmitoylguanine.

9-(3-Azido-2,3-dideoxy-5-O-acetyl-β-D-erythro-pentofuranosyl)-N-2-palmitoylguanine. (2.20g, 3.83 mmoles) was dissolved in 150 mL of saturated methanolic ammonia. The solution was stirred 18 h at room temperature and the solvent then removed by rotary evaporation. The remaining solids were dissolved in 100 mL of EtOH:conc. ammonium hydroxide (9:1) at reflux, filtered and stirred 1 h in an ice bath. The precipitated solids were collected by filtration, washed with 10 mL portions of prechilled methanol and acetone and dried overnight in a vacuum oven at 65°C to afford 756 mgs (67.5%) of 9-(3-azido-2,3-dideoxy-β-D-erythro-pentofuranosyl)-guanine as a white solid: mp > 300°C; 1H NMR (d6-DMSO) δ 2.25-2.28 (m,2H,C-2'H), 3.20 (m,2H,C-5'H), 3.82 (q,J=4.50Hz,1H,C-4'H0, 4.54 ((m,1H,C-3'H), 5.08 (t,J=5.35Hz,1H,OH), 6.07(t,J=6.5Hz,12H,C-1'H), 6.46 (broad s,2H,NH2), 7.92(s,1H,c-8H) and 10.60 (broad s,1H,NH); Elemental Analysis calcd (as 1/2 hydrate) C, 39.87; H, 4.35; N, 37.19. Found C,39.90; H, 4.33; N, 37.21.

## Pharmaceutical Formulations

In the following formulation Examples, the "Active Ingredient" may be 9-(3-azido-2,3-dideoxy-β-D-erythro-pentofuranosyl)guanine or a salt, ester or other physiologically functional derivative thereof.

## Example 2

### Tablet Formulations

The following formulations A, B and C are prepared by wet granulation of the ingredients with a solution of povidone, followed by addition of magnesium stearate and compression.

Formulation A

|  |  | mg/tablet | mg/tablet |
|---|---|---|---|
| (a) | Active ingredient | 250 | 250 |
| (b) | Lactose B.P. | 210 | 26 |
| (c) | Povidone B.P. | 15 | 9 |
| (d) | Sodium Starch Glycollate | 20 | 12 |
| (e) | Magnesium Stearate | 5 | 3 |
|  |  | 500 | 300 |

Formulation B

|  |  | mg/tablet | mg/tablet |
|---|---|---|---|
| (a) | Active ingredient | 250 | 250 |
| (b) | Lactose | 150 | - |
| (c) | Avicel PH 101 | 60 | 26 |
| (d) | Povidone B.P. | 15 | 9 |
| (e) | Sodium Starch Glycollate | 20 | 12 |
| (f) | Magnesium Stearate | 5 | 3 |
|  |  | 500 | 300 |

Formulation C

|  | mg/tablet |
|---|---|
| Active ingredient | 100 |
| Lactose | 200 |
| Starch | 50 |
| Povidone | 5 |
| Magnesium Stearate | 4 |
|  | 359 |

The following formulations, D and E, are prepared by direct compression of the admixed ingredients. The lactose in formulation E is of the direct compression type (Dairy Crest - "Zeparox").

Formulation D

|  | mg/tablet |
|---|---|
| Active ingredient | 250 |
| Pregelatinized Starch NF15 | 150 |
|  | 400 |

Formulation E

|  | mg/tablet |
|---|---|
| Active ingredient | 250 |
| Lactose | 150 |
| Avicel | 100 |
|  | 500 |

Formulation F (Controlled Release Formulation)

The formulation is prepared by wet granulation of the ingredients (below) with a solution of povidone followed by the addition of magnesium stearate and compression.

|  |  | mg/tablet |
|---|---|---|
| (a) | Active ingredient | 500 |
| (b) | Hydroxypropylmethylcellulose (Methocel K4M Premium) | 112 |
| (c) | Lactose B.P. | 53 |
| (d) | Povidone B.P. | 28 |
| (e) | Magnesium Stearate | 7 |
|  |  | 700 |

Drug release takes place over a period of about 6-8 hours and is complete after 12 hours.

Exemple 3

Capsule Formulations

Formulation A

A capsule formulation is prepared by admixing the ingredients of Formulation D in Example 2 above and filling into a two-part hard gelatin capsule. Formulation B (infra) is prepared in a similar manner.

Formulation B

|  |  |  | mg/capsule |
|---|---|---|---|
| (a) | Active ingredient | | 250 |
| (b) | Lactose B.P. | | 143 |
| (c) | Sodium Starch Glycollate | | 25 |
| (d) | Magnesium Stearate | | 2 |
| | | | 420 |

Formulation C

|  |  | mg/capsule |
|---|---|---|
| (a) | Active ingredient | 250 |
| (b) | Macrogol 4000 B.P. | 350 |
| | | 600 |

Formulation D

|  | mg/capsule |
|---|---|
| Active ingredient | 250 |
| Lecithin | 100 |
| Arachis Oil | 100 |
| | 450 |

Capsules of formulation D are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients a, b and c using an extruder, followed by spheronization of the extrudate and drying. The dried pellets are then coated with release-controlling membrane (d) and filled into a two-piece, hard gelatin capsule.

|  |  | mg/capsule |
|--|--|:--:|
| (a) | Active ingredient | 250 |
| (b) | Microcrystalline Cellulose | 125 |
| (c) | Lactose B.P. | 125 |
| (d) | Ethyl Cellulose | 13 |
|  |  | 513 |

## Example 4

Injectable Formulation

### Formulation A

| | |
|--|--|
| Active ingredient | 0.200 g |
| Hydrochloric acid solution, 0.1 M, or | |
| Sodium hydroxide solution, 0.1 M  q.s. to pH | 4.0 to 7.0 |
| Sterile water | q.s. to 10 mL |

The active ingredient is dissolved in most of the water (35°C-40°C) and the pH adjusted to between 4.0 and 7.0 with the hydrochloric acid or the sodium hydroxide as appropriate. The batch is then made up to volume with the water and filtered through a sterile micropore filter into a sterile 10 mL amber glass vial (type 1) and sealed with sterile closures and overseals.

### Formulation B

| | |
|--|--|
| Active ingredient | 0.125 |
| Sterile, pyrogen-free, pH 7 phosphate | |
| Buffer, | q.s. to 25 mL |

## Example 5

Intramuscular injection

| | |
|--|--|
| Active ingredient | 0.20 g |
| Benzyl Alcohol | 0.10 g |
| Glycofurol 75 | 1.45 g |
| Water for Injection | q.s. to 3.00 mL |

The active ingredient is dissolved in the glycofurol. The benzyl alcohol is then added and dissolved, and water added to 3 mL. The mixture is then filtered through a sterile micropore filter and sealed in sterile 3 mL amber glass vials (type 1).

Example 6

Syrup

| | | |
|---|---|---|
| Active ingredient | | 0.25 g |
| Sorbitol Solution | | 1.50 g |
| Glycerol | | 2.00 g |
| Sodium Benzoate | | 0.005 g |
| Flavor, Peach 17.42.3169 | | 0.0125 mL |
| Purified Water | q.s. to | 5.00 mL |

The active ingredient is dissolved in a mixture of the glycerol and most of the purified water. An aqueous solution of the sodium benzoate is then added to the solution, followed by addition of the sorbitol solution and finally the flavor. The volume is made up with purified water and mixed well.

Formulation B

| | | |
|---|---|---|
| Active ingredient | | 0.125 g |
| Sterile, pyrogen-free, pH 7 phosphate buffer, | q.s. to | 25 mL |

Example 7

Suppository

| | mg/suppository |
|---|---|
| Active ingredient | 250 |
| Hard Fat, B.P. (Witepsol H15 - Dynamit NoBel) | 1770 |
| | 2020 |

One-fifth of the Witepsol H15 is melted in a steam-jacketed pan at 45°C maximum. The active ingredient is sifted through a 200 μM sieve and added to the molten base with mixing, using a Silverson fitted with a cutting head, until smooth dispersion is achieved. Maintaining the mixture at 45°C, the remaining Witepsol H15 is added to the suspension and stirred to ensure a homogenous mix. The entire suspension is passed through a 250 μM stainless steel screen and, with continuous stirring, is allowed to cool to 40°C. At a temperature of 38°C to 40°C, 2.02 g of the mixture is filled into suitable, 2 mL plastic molds. The suppositories are allowed to cool to room temperature.

Example 8

Pessaries

| | mg/pessary |
|---|---|
| Active ingredient | 250 |
| Anhydrate Dextrose | 380 |
| Potato Starch | 363 |
| Magnesium Stearate | 7 |
| | 1000 |

The above ingredients are mixed directly and pessaries prepared by direct compression of the resulting mixture.

Example 9

Antiviral Activity Against Hepatitis B Virus (HBV)

i) Determination of anti-HBV activity was carried out by testing the ability of a compound to prevent replication of duck HBV in vitro, in the manner described by Tuttleman, Pugh and Summers (J. Virol., 58:17-25, 1986). Duck hepatocytes were obtained and placed in culture, and infected with duck HBV. Three days after infection, the infected cells were exposed to various concentrations of the test compound for an additional period of eight days. After this exposure, DNA was extracted from each culture of infected cells and compound, and the amount of viral DNA was specifically determined and compared with that obtained from similar cultures lacking the test compound.

The compound 9-(3-azido-2-3-dideoxy-β-D-erythro-pentofuranosyl)-guanine was tested as described above and found to be active at 1 μM.

ii) Anti-HBV activity was determined using the method described by Korba B.E. and Milman G., Antiviral Research 1991, V-1 15, pp 217-228. The human HBV producer cell line of HepG2, 2.2.15, described and characterized by Sells et al., PNAS 84:1005, 1987 and J. Virol. 62:2836, 1988, has been shown to share many characteristics of the HBV chronically infected hepatocyte. It is infectious as demonstrated by the ability to cause disease in chimpanzees. This cell line was utilized in vitro to identify compounds with anti-HBV activity.

To test compounds for antiviral activity, monolayer cultures were treated with compound, 50-200 μM, for ten days. Supernatant media containing extracellular virion DNA (Dane particles) were harvested on days three, six and ten, treated with proteinase K (1 mg/mL) and sodium dodecyl sulfate (1%), and incubated at 50°C for one hour. DNA was extracted with equal volumes of phenol followed by chloroform and then precipitated by ammonium acetate and propanol. The DNA precipitate was dissolved and collected on nitrocellulose using the procedure of Schleicher and Schuell (S & S, 10 Optical Ave., Keene, NH 03431, Publication #700, 1987), and treated as described by Southern, J. Mol. Biol. 98:503, 1975. Cells were harvested, and the intracellular DNA was obtained after cell lysis with guanidine isothiocyanate. The intracellular DNA was handled in the same manner as the extracellular DNA. After precipitation by ammonium acetate and propanol, the intracellular DNA precipitate was dissolved, cut by restriction endonuclease, Hind III, applied to agarose gel and then treated as described by Southern to determine the quantity of replicative intermediate forms. The antiviral effect of the drug was determined by measuring at least a 100-fold reduction of the amount of Dane particles extruded into the culture medium and a similar decrease in the intracellular replicative intermediates.

HBV DNA IN CULTURE MEDIUM

(PG/mL)

|  |  | Day 0 | Day 3 | Day 6 | Day 10 |
|---|---|---|---|---|---|
|  | Untreated Cells | 62 | 84 | 87 | 93 |
| 9-(3-Azido-2,3-dideoxy-$\beta$-D-erythropento-furanosyl) guanine | Treated Cells | 96 | 80 | 45 | 20 |

Cell toxicity was measured using D98 cells in a microtiter based assay. Cells were seeded into wells of a 96-well plate at a density of 5000 per well. These cultures were grown at 36°C and 100% humidity for 72 hours. Total cell counts were compared between replicate test and control cultures. Controls showed at least 2.5 cell doublings. The cells were enumerated by a viral stain (neutral red) to asses the number of viable cells. Cell growth of D98 cells subjected to 9-(3-azido-2,3-dideoxy-β-D-erythro-pentofuranosyl)guanine was 91% of controls at 100μM.

**Claims**

1. Use of the compound 9-(3-azido-2,3-dideoxy-β-D-erythro-pentofuranosyl)guanine in the manufacture of a medicament for the treatment or prophylaxis of hepatitis B viral infections.

2. Use of a physiologically acceptable salt, ester or other functional derivative of 9-(3-azido-2,3-dideoxy-β-D-erthro-pentofuranosyl)guanine in the manufacture of a medicament for the treatment or prophylaxis of hepatitis B viral infections.

3. Use of the compound or a physiologically acceptable salt, ester or other functional derivative thereof as claimed in claim 1 or 2 wherein the medicament is in a form adapted for oral administration.

4. Use of the compound or a physiologically acceptable salt, ester or other functional derivative thereof according to claim 3 wherein the medicament is in the form of a tablet or capsule.

5. Use of the compound or a physiologically acceptable salt, ester or other functional derivative thereof as claimed in claim 1 or 2 wherein the medicament is in a form adapted for parenteral administration.

6. Use of the compound or a physiologically acceptable salt, ester or other functional derivative thereof according to claim 5 wherein the medicament is in the form of an aqueous or non-aqueous sterile solution.

7. Use of the compound or a physiologically acceptable salt, ester or other functional derivative thereof according to any one of the preceding claims wherein the medicament is in a unit dosage form containing 10 to 700 mg of the compound or a physiologically acceptable salt or ester thereof.

8. Use of the compound or a physiologically acceptable salt, ester or other functional derivative thereof according to any one of the preceding claims wherein the medicament is in a unit dosage form for administration to achieve an effective dose in the range of 2 to 60 mg of 9-(3-azido-2,3-dideoxy-β-D-erythro-pentofuranosyl)guanine or a physiologically acceptable salt, ester or other functional derivatives thereof per kilogram of body weight of recipient per day.

| | **European Patent Office** | **EUROPEAN SEARCH REPORT** | **Application Number** |
|---|---|---|---|
| | | | EP 92 30 2373 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| D,X | EP-A-0 217 580 (THE WELLCOME FOUNDATION, LTD) * Page 5, paragraph 1; page 10, paragraphs 3,4; pages 25-30; claims 1,6-8,11,16,50-55 * --- | 1-8 | A 61 K 31/70 |
| D,X | EP-A-0 286 825 (MAX-PLANCK-GESELLSCHAFT ZUR FÖRDERUNG DER WISSENSCHAFTEN e.V.) * Whole document, especially claims 1,3,17 * --- | 1-8 | |
| Y | BIOCHEMICAL AND BIOPHYSICAL RESEARCH COMMUNICATIONS, vol. 145, no. 3, 30th June 1987, pages 1080-1086; M. BABA et al.: "Selective inhibition of human immunodeficiency virus (HIV) by 3'-azido-2',3'-dideoxyguanosine in vitro" * Whole article * --- | 1-8 | |
| Y | EP-A-0 311 108 (STICHTING REGA V.Z.W.) * Whole document, especially claim 2 * --- | 1-8 | **TECHNICAL FIELDS SEARCHED (Int. Cl.5)** A 61 K |
| Y | BIOCHEMICAL AND BIOPHYSICAL RESEARCH COMMUNICATIONS, vol. 156, no. 3, 15th November 1988, pages 1144-1151; S. SUZUKI et al.: "Inhibition of duck hepatitis B virus replication by purine 2',3'-dideoxynucleosides" * Whole document * ----- | 1-8 | |

The present search report has been drawn up for all claims

| Place of search | Date of completion of the search | Examiner |
|---|---|---|
| THE HAGUE | 15-06-1992 | ORVIZ DIAZ P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

14